# EUROPEAN PATENT APPLICATION

(11) **EP 2 130 618 A1**
(43) Date of publication of application: **09.12.2009**
(21) Application number: 09006738.0
(22) Date of filing: 19.05.2009
(51) Int. Cl.: B09B 3/00, C02F 3/28, C02F 11/04

(54) **Apparatus for anaerobic stabilization of the organic fraction of urban solid waste**

(30) Priority: 26.05.2008 IT TO20080394
(71) Applicant: VM Press S.r.l., 15076 Ovada (Al) (IT)
(72) Inventor: Vincenti, Enrico, 06012 Citta di Castello (PG) (IT); Ceccaroni, Luciano, 47900 Rimini (IT)
(74) Representative: Spandonari, Carlo

(57) **Abstract**

The apparatus (10) comprises a mixing bin (22) in which the organic material to be processed is preliminarly homogenized, and a digester station (36) which comprises at least two digester bins (40, 42) which are selectively connectable in series or in parallel for receiving organic material from the mixing bin (22) and releasing it after a predetermined digestion period.

## Description

The present invention relates to an apparatus for anaerobic stabilization of the organic fraction of urban solid waste

As known, the urban solid waste is an eterogenous material which is very difficult to process. Such material tipically contains 40% of humid, organic biomass and 60% of solid material. It is known, e.g., from Italian Patent No. 1 255 197, to press the urban solid waste under very high pressures in order to separate by extrusion the humid biomass, which takes the shape of a gel, from the "dry" fraction, which can be recycled, e.g., as fuel.

After the separation, the gel of organic material is subjected to a process of anaerobic digestion in a bin, where it is degraded in absence of oxygen by various groups of micro-organisms, which mainly comprise anaerobic bacteria and facultative bacteria, thanks to which a series of reactions starts which transform the organic substance into intermediate compounds (mainly acetic acid, carbon dioxide and hydrogen), which are subsequently used as a substrate for the production of methane.

A conventional digester bin has a cylindrical side wall and a frustoconical bottom which is closed at its lower end by a valve that can be opened to release the material at the end of the digestion process. Since it is essential to increase the volume of the bin but, at the same time, it is necessary to reduce its vertical size, usually the bin is shaped with a relatively large diameter and a relatively short height, with a ratio volume/diameter that is tipically in the range 150 to 200 (although this ratio should be given in m², it is conventionally given as a factor without unit of measurement because it refers to a substantially cylindrical shape, whereby the resulting geometrical shape is univically defined.

However, as known to the person skilled in the art, the above shape affects the efficiency of the apparatus, because the larger the diameter of the bin, the higher the risk that isolated pokets of inert material form in the peripheral areas of the bin, which pokets are not involved in the fermentation process and remain blocked in the bin. Furthermore, preferential courses are liable to form in the material stored in the bin, through which the fresh material loaded into the bin flows, thereby preventing this fresh material from mixing uniformly with the whole mass and, consequently, affecting the homogeneity of the final product.

Furthermore, it would be desirable to provide an apparatus having a higher versatility and, particularly, which allows a higher number of process parameters to be controlled and adjusted in order to optimize the efficiency of the process.

Therefore, it is a main object of the present invention to provide an apparatus for anaerobic stabilization of the organic fraction of urban solid waste, which has a higher efficiency, a higher versatility and a higher controllability with respect to the known apparatuses.

It is a further object of the invention to provide an apparatus smaller in size than the known apparatuses, for equal loading capacity.

The above object and other advantages, which will better appear from the following description, are achieved by the apparatus having the features recited in claim 1, while the dependent claims state other advantageous, though secondary, features of the invention.

The invention will be now described in more detail, with reference to a few preferred, non-exclusive embodiments shown by way of non-limiting example in the attached drawings, wherein:
Fi. 1 is a view in side elevation of the apparatus according to the invention;
Fig. 2 is a view to an enlarged scale of a first detail of Fig. 1;
Fig. 3 is a view to an enlarged scale of a second detail of Fig. 1;
Fig. 4 is a view similar to Fig. 3 but showing an alternative embodiment of the apparatus according to the invention.

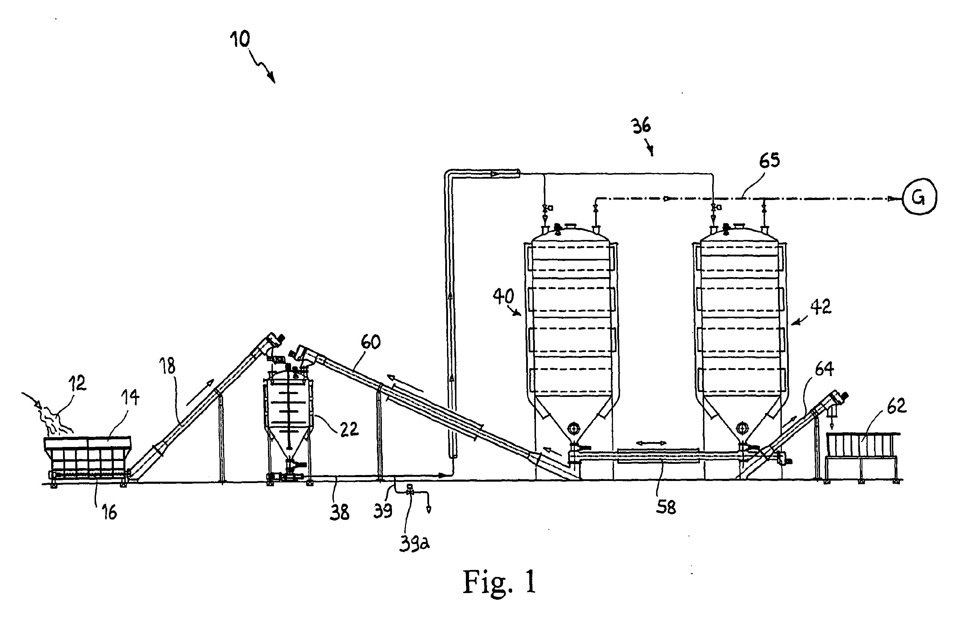

With initial reference to Figs. 1-3, an apparatus 10 for anaerobic stabilization of the organic fraction of urban solid waste is particularly, though non-exclusively, suitable for processing gel of organic material derived by extrusion under pressure of urban solid waste, preferably by means of a machine of the type described in IT 1 255 197.

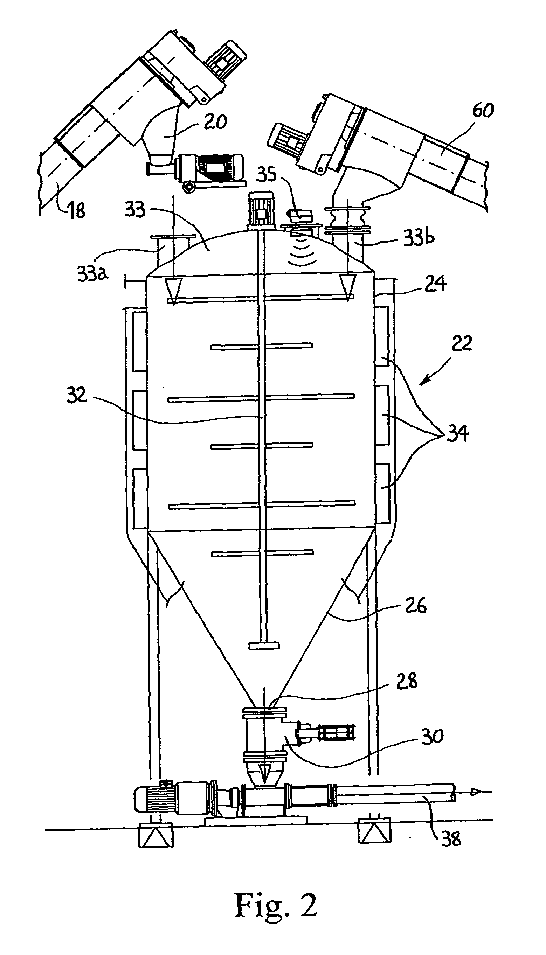

In apparatus 10, gel 12 is loaded into a closed hopper 14 in which the material can be stored for a predetermined period. A drawing auger 16 is arranged at the bottom of hopper 14 for conveying gel 12 from the hopper to a feeding auger 18 slanting upwards. A bladed mincer 20 (Fig. 2) is arranged at the top of feeding auger 18 for mincing the material received therefrom.

The material minced by bladed mincer 20 is fed to a mixing bin 22 by gravity, as shown in detail in Fig. 2. Mixing bin 22 comprises a cylindrical wall 24 made of stainless steel and having a frustoconical bottom 29 tapering downwards. Frustoconical bottom 26 has an outlet 28 at its lower end, which is closed by a guillotine valve 30. A motorized, bladed mixer 32 is arranged within mixing bin 22. The upper end of mixing bin 22 is closed by a cover 33 having an inlet 33a aligned below the delivery section of bladed mincer 20 to receive material from it, as well as a recycle mouth 33b. The mixing bin is surrounded by a triad of hollow, heating rings 34 into which hot fluid is circulated, which fluid is preferably drawn by a boiler (not shown) which uses biogas generated by the apparatus. Mixing bin 22 is also provided with a sensor 35 for measuring the level of the material in the bin.

The material evacuated from mixing bin 22 is conveyed to a digester station 36 by an auger which is housed in a heated, feeding duct 38 for minimizing the loss of heat. The feeding duct also is preferably heated by drawing hot fluid from the boiler. A branch 39 of feeding duct 38 is provided with a normally closed, solenoid valve 39a which may be opened to empty the bin.

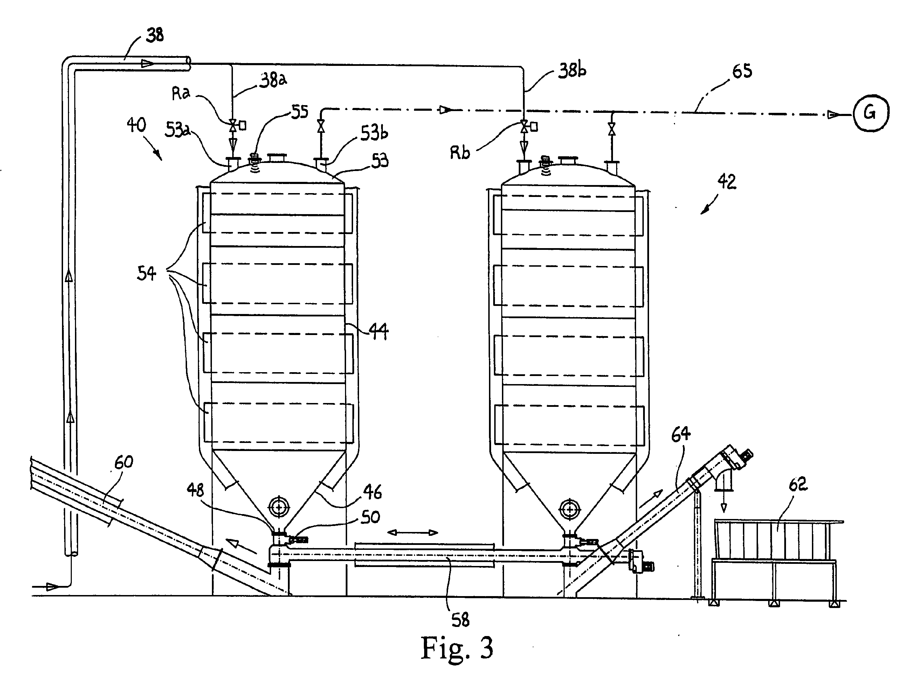

Digester station 36 (Fig. 3) comprises a pair of digester bins 40, 42 which are larger in size than mixing bin 22. The digester bins each comprise a cylindrical wall 44 made of stainless steel and having a frustoconical bottom 46 provided with an outlet 48 at its lower end, which is closed by a guillotine valve 50. The upper end of the digester bin is closed by a cover 53 having an inlet 53a and an outlet 53b for extraction of biogas. Both digester bins 40, 42 are surrounded by a triad of hollow heating rings 54, into which hot fluid drawn by the boiler is circulated, and are provided with respective level sensors 55.

Advantageously, both digester bins 40, 42 are smaller in volume than conventional digester bins, e.g., in the range 100-500 m³, and have an elongated shape, with a ratio volume/diameter in the range 15-50, preferably 35. This allows the material to flow freely in the vertical direction from inlet 53a to outlet 48 and mixing with the whole material contained in the bin, without requiring any auxiliary mixer.

Digester bins 40, 42 are both connected to a piping which allows them to operate both in series and in parallel, with high versatility in controlling the digestion process and consequent optimization of the efficiency of the apparatus. In particular, digester bins 40, 42 are fed by respective branches 38a, 38b of feeding duct 38, which are provided with respective valves Ra, Rb. The outlets of the bins are interconnected by an insulated, extraction auger 58 which is selectively operable in both directions for the scopes which will be clarified in the prosecution of this description. Preferably, extraction auger 58 is relatively large in diameter, whereby the material can be completely extracted from it while preventing jamming. In particular, the ratio between the diameter of extraction auger 58 and the diameter of digester bins 40, 42 is in the range 1/20-1/10.

A fraction of the material extracted from the bins can be recycled via an insulated, recycle auger 60 which connects outlet 48 of first digester bin 40 to recycle mouth 33b of mixing bin 22. The remaining fraction of the material extracted from the bins is discharged into a container 62 via an outlet auger 64. The biogas-draining mouths are both connected to a biogas-draining duct 65 connected to a gasometer G (which is diagrammatically shown in Figs. 1 and 3). The biogas is then conveyed from gasometer G to a conventional cogeneration apparatus (not shown).

In operation, gel 12 in hopper 14 is periodically fed to mixing bin 22 by extraction auger 16 and feeding auger 18. Before entering the mixing bin, the material is minced by bladed mincer 20, in order to enhance the contact between the microbic population and the substratum to be processed.

In mixing bin 22, the material is homogenized by bladed mixer 32 and heated up to a temperature preferably in the range 35 to 55 °C by heating rings 34. As mentioned above, a measured amount of material which has already been processed may be recycled into the mixing bin through recycle mouth 33b, according to mixing ratios which depend on the chemical and organoleptic characteristics of the material under processing. Therefore, the mixer allows a homogeneous material, which has been already heated up to a predetermined temperature, to be fed to digester station, thereby optimizing the output of the digestion process.

By opening guillotine valve 30, the material is fed from mixing bin 22 to the digester station via heated duct 38. As mentioned above, the apparatus according to the invention may be operated in various modes, e.g., by using only one of the digester bins 40, 42 when the daily load is lower then a nominally expected load, or by using both the digester bins in series mode, in parallel mode, or in a hybrid series/parallel mode. The operative mode of the apparatus may be chosen on the basis of operative parameters of the digestion system which are well known to the person skilled in the art, e.g., in order to prevent phenomena of accumulation of volatile fat acids, with consequent reduction of PH in the digester bins.

In case of high amounts of material to be processed, it will be generally convenient to use both digester bins in parallel. In this case, the material is simultaneously fed from mixing bin 22 to both the digester bins by opening both valves Ra, Rb. After a predetermined fermentation period, the material in the bins is evacuated by opening guillotine valves 50, and is drawn out by operating extraction auger 58 rightwards (in Fig. 1) and outlet auger 64. As mentioned above, a fraction of the material can be recycled by operating extraction auger 58 leftwards (in Fig. 1) and recycle auger 60.

In series mode, the organic material, in a first step, is only fed to first digester bin 40, while valve Rb leading to second digester bin 42 is closed. After a predetermined digestion period, the material is fed to mixing bin 22 via recycle auger 60 and, from mixing bin 22 to second digester bin 42, while valve Ra leading to the first bin is closed.

In any case, during the digestion process, the material is heated up by rings 54 to a temperature which is preferably in the range 35 to 55 °C.

Of course, the various parts of the apparatus are controlled by a control unit (not shown) which is conventionally programmable for automatically managing the above process.

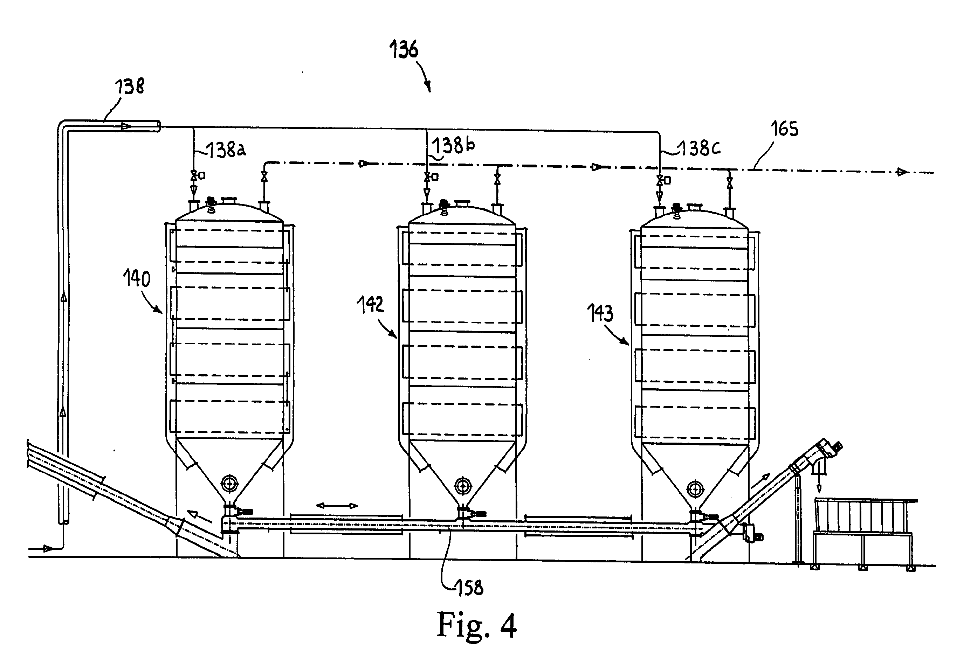

Fig. 4 shows an alternative embodiment of the invention, wherein digester station 136 comprises three bins 140, 142, 143 which are connected likewise the previous embodiment. In particular, the three bins are fed via respective branches 138a, 138b, 138c of feeding duct 138 and are connected to feed the digested material to an extraction auger 158, which is operable in both directions and is shaped to receive the material from the three outlets of the digester bins, and the biogas to a biogas-draining duct 165.

As the person skilled in the art will immediately understand, the apparatus of the invention allows high amounts of organic material to be processed effectively in a relatively small-sized apparatus. In fact, contrary to the common practice, the apparatus comprises a plurality of digester bins having an elongated shape and interconnected in series or in parallel, rather than a single short, large bin which, as mentioned above, may be affected by drawbacks in terms of efficiency. The connection in series/parallel of the bins makes the apparatus very versatile and adjustable, e.g., depending on the characteristics and on the amount of material to be processed. A further advantage of the apparatus according to the invention is that, when it is fed with an organic gelatinous material separed from urban solid waste, a lower amount of dilution water is required for processing the material with respect to a conventional apparatus, and in certain cases no dilution water is required. Preferably, the percentage of dilution water shoud be such that the mix fed to the digester station has an amount of dry material (TS) in the range 16% to 35%.

A preferred embodiment of the invention has been described herein, but of course many changes may be made by a person skilled in the art within the scope of the claims. In particular, the digester station may comprise a higher number of bins, e.g., four, five, or more bins connected likewise the described embodiments. Moreover, certain components of the apparatus may be replaced by other components capable of performing the same function in a similar way. For example, the bladed mincer 20 may be replaced by other mincing means such as cutting mincers, and the like. Moreover, although in the preferred embodiments the bins are made of stainless steel, they could also be made of other similar, or suitable, material, e.g., iron. Nevertheless, the number of heating rings used for heating the bins may be different from what described. Moreover, different heating means may be used in lieu of the rings, e.g., continuous cylinders surrounding the full height of the bin.

## Claims

1. An apparatus (10) for anaerobic stabilization of organic material (12) derived from urban solid waste, **characterized in that** it comprises:
- a mixing bin (22), in which the organic material to be processed is preliminarly homogenized, and
- a digester station (36) comprising at least two digester bins (40, 42) which are selectively connectable in series or in parallel for receiving organic material from said mixing bin (22) and releasing it after a predetermined digestion period.

2. The apparatus of claim 1, **characterized in that** said mixing bin (22) is provided with a motorized bladed mixer (32).

3. The apparatus of claim 1 or 2, **characterized in that** said digester bins (40, 42) each comprise a substantially cylindrical, elongated wall (44) having a ratio volume/diameter in the range 15 to 50.

4. The apparatus of claim 1, **characterized in that** said ratio volume/diameter of each of said digester bins is 35.

5. The apparatus of claim 4, **characterized in that** the volume of each of said digester bins (40, 42) is in the range 100 to 500 m³.

6. The apparatus of claim 1, **characterized in that** said mixing bin (22) is provided with heating means (34).

7. The apparatus of any of claims 1, **characterized in that** said digester bins (40, 42) are provided with heating means (54).

8. The apparatus of any of claims 6 or 7, **characterized in that** said heating means comprise hollow heating rings in which hot fluid is circulated.

9. The apparatus of claim 1, **characterized in that** it comprises mincing means (20) arranged for mincing the material entering the mixing bin.

10. The apparatus of claim 1, **characterized in that** said digester bins (40, 42) are connected to receive material from said mixing bin (22) via a heated feeding duct (38).

11. The apparatus of any of claims 1 to 10, **characterized in that** it comprises a recycle auger (60) which is connected to recirculate at least a fraction of the material released from the digester bins (40, 42) into the mixing bin (22).

12. The apparatus of claim 11, **characterized in that** said digester bins (40, 40) are interconnected via an extraction auger (58) which is selectively operable in both directions to transfer the digested material to said recycle auger (60) or to outlet means (64) leading to a collecting area (62).

13. A process for the anaerobic stabilization of organic material (12) derived from urban solid waste, **characterized in that** it comprises the steps of:
- homogenizing the organic material in a heated mixing bin (22) provided with mixing means (32), and
- stocking the homogenized material drawn from the mixing bin (22) for a predetermined period in a digester station provided with at least two heated digester bins (40, 42) interconnectable in series or in parallel.

14. The process of claim 13, **characterized in that** a fraction of the material digested in said digester station is recycled into said mixing bin (22).

15. The process of claim 13 or 14, **characterized in that** the material in the digester bin is diluted with a low percentage of water such that the mix in the digester has an amount of dry material in the range 16% to 35%.
